(19) Europäisches Patentamt

European Patent Office

Office européen des brevets

(11) Numéro de publication: **0 498 726 B1**

(12) **FASCICULE DE BREVET EUROPEEN**

(45) Date de publication de fascicule du brevet: **03.05.95** (51) Int. Cl.⁶: **C07C 11/02**, C07C 1/20,
B01J 8/24, B04C 5/081,
B04C 5/103

(21) Numéro de dépôt: **92400301.5**

(22) Date de dépôt: **05.02.92**

(54) Procédé et dispositif pour la conversion d'un composé oxygéné en hydrocarbures oléfiniques.

(30) Priorité: **07.02.91 FR 9101519**

(43) Date de publication de la demande:
**12.08.92 Bulletin 92/33**

(45) Mention de la délivrance du brevet:
**03.05.95 Bulletin 95/18**

(84) Etats contractants désignés:
**DE GB IT NL**

(56) Documents cités:
**US-A- 4 197 418**

(73) Titulaire: **INSTITUT FRANCAIS DU PETROLE**
**4, avenue de Bois Préau**
**F-92502 Rueil-Malmaison (FR)**

(72) Inventeur: **Hugues, François**
**10 Chemin du Clos Challans,**
**Charly**
**F-69390 Vernaison (FR)**
Inventeur: **Vuillemot, Daniel**
**27 rue du l'Haye**
**F-69230 St Genis Laval (FR)**
Inventeur: **Burzynski, Jean-Pierre**

**25, Rue du Brulet**
**F-69005 Lyon France (FR)**
Inventeur: **Galtier, Pierre**
**Centre Hospitalier Mont Salamon,**
**BP 12**
**F-38209 Vienne Cédex (FR)**
Inventeur: **Gauthier, Thierry**
**13, Allée des Muses**
**F-69230 Saint Genis Laval (FR)**

(74) Mandataire: **Andreeff, François et al**
**INSTITUT FRANCAIS DU PETROLE**
**4, avenue de Bois-Préau**
**F-92502 Rueil-Malmaison (FR)**

Rank Xerox (UK) Business Services
(3.10/3.09/3.3.3)

EP 0 498 726 B1

# Description

L'invention concerne un procédé de conversion catalytique, en lit entraîné, d'une charge comprenant au moins un composé oxygéné et plus spécialement au moins un alcool tel que le méthanol et/ou au moins un éther-oxyde tel que le diméthyléther, et un dispositif, pour la mise en oeuvre de ce procédé comprenant au moins un séparateur cyclonique à co-courant permettant la séparation très rapide des particules solides et des gaz.

Ce procédé s'applique plus particulièrement à la production d'hydrocarbures oléfiniques, notamment à usage pétrochimique, comprenant une majeure partie de composés ayant de 2 à 4 atomes de carbone dans leur molécule, en présence d'un catalyseur comme une zéolithe circulant dans un réacteur à lit entraîné.

Une utilisation particulière donnée ici à titre d'exemple est la conversion catalytique du méthanol en hydrocarbures riches en éthylène et/ou en propylène.

L'arrière plan technologique est illustré par le brevet US-A-4 197 418.

Un des facteurs essentiels à la production sélective d'oléfines légères, et plus particulièrement d'éthylène et/ou de propylène par conversion du méthanol, est la maîtrise du temps de contact entre la charge et le catalyseur. Ce contact entre la charge et le catalyseur doit être à la fois court et uniforme (ou régulier) dans le temps, ce qui impose une rapidité et une uniformité de la mise en contact entre les grains de catalyseur et la charge gazeuse ou liquide. Il est donc important de bien contrôler, en particulier, la quantité de charge introduite en liaison avec la quantité de catalyseur avec lequel elle entre en contact, ainsi que de garder constant le temps de contact entre la charge et le catalyseur de manière à obtenir la meilleure sélectivité possible en le ou les composés éthyléniques souhaités. En l'absence d'un tel contrôle la tendance de la réaction est de conduire à une large gamme d'hydrocarbures saturés et insaturés allant généralement du méthane à des hydrocarbures ayant jusqu'à 10 atomes de carbone dans leur molécule ou plus, tels que ceux constituant habituellement la coupe essence.

Dans les procédés de conversion du méthanol, utilisant la technique du lit entraîné, décrits dans l'art antérieur, on insiste le plus souvent sur l'importance de l'optimisation et du contrôle du temps de contact entre les particules catalytiques et la charge. Ce temps de contact doit être, comme cela est par exemple mentionné dans le brevet US-A-4229608, relativement court. Cependant on ne mentionne pas l'utilisation d'un système de séparation adapté à permettre une séparation efficace et rapide des particules solides et des gaz contenant les produits de la réaction.

L'association de conditions opératoires bien choisies et d'une séparation ultra-rapide des particules catalytiques et des produits gazeux incluant les produits de la conversion permet en particulier d'améliorer sensiblement la sélectivité en le ou les composés éthyléniques souhaités, ce qui n'était pas le cas dans les réalisations décrites dans l'art antérieur telle que par exemple celle décrite dans le brevet US-A-4046825.

De façon plus précise la présente invention concerne un procédé de conversion catalytique d'une charge contenant au moins un composé oxygéné, en hydrocarbures oléfiniques riches en composés ayant de 2 à 4 atomes de carbone dans leur molécule, comprenant la conversion en lit entraîné de ladite charge, dans une zone réactionnelle de conversion de forme allongée, dans des conditions appropriées, en présence d'un catalyseur sous forme de particules solides, ledit procédé comportant une étape d'alimentation, dans une zone située à proximité d'une première extrémité de ladite zone réactionnelle, en au moins un solide sous forme de particules contenant des particules catalytiques et en au moins un fluide d'entraînement, une étape d'introduction, et, dans le cas d'une charge au moins en partie liquide, de pulvérisation de ladite charge dans une zone d'introduction située en aval, dans le sens du déplacement des particules solides, de la zone d'introduction desdites particules solides, une étape de mise en contact desdites particules solides et de ladite charge dans une zone située à proximité de la première extrémité, une étape de circulation des particules solides et de la charge dans la zone de réaction au cours de laquelle on effectue la conversion de ladite charge et on désactive au moins en partie les particules solides catalytiques par dépôt de coke sur celles-ci, une étape de séparation des particules solides et des gaz contenant les produits de la conversion, au moins partielle, de ladite charge, dans une zone de séparation située à proximité d'une deuxième extrémité de la zone de réaction à l'opposé de ladite première extrémité, une étape de régénération, dans au moins une zone de régénération, d'au moins une partie des particules solides catalytiques, au moins en partie désactivées et une étape de recyclage des particules solides catalytiques, au moins en partie régénérées, dans une zone de recyclage à proximité de ladite première extrémité caractérisé en ce que la séparation des particules solides et des gaz contenant les produits de la réaction est effectuée dans un séparateur cyclonique à co-courant.

Dans une forme particulière de réalisation le séparateur cyclonique à co-courant comprend au moins un moyen d'introduction d'au moins un fluide permettant d'effectuer simultanément le strippage des particules solides. Ce fluide est habituellement un gaz choisi parmi les gaz employés de façon classique par les hommes du métier pour effectuer un tel strippage. Ce gaz sera par exemple de la vapeur d'eau ou un gaz inerte tel que par exemple de l'azote.

Dans ce type de cyclone à co-courant on peut, contrairement au cas des cyclones à rebours, en plaçant l'entrée interne de la phase légère L1 (essentiellement gazeuse) assez près de l'entrée (à une distance inférieure à la longueur (Lc) du cyclone à rebours) du mélange M1 (contenant les particules solides formant une phase dense D1 et les gaz formant une phase légère L1) et en contrôlant la circulation de la phase légère dans le séparateur, obtenir une séparation rapide des phases tout en conservant une bonne efficacité de la collecte de la phase dense D1 et en ayant une distribution de temps de séjour de la phase légère acceptable.

Dans une forme préférée de réalisation de la présente invention on utilisera un séparateur cyclonique à co-courant particulier permettant d'effectuer très rapidement la séparation de la phase dense D1 et de la phase légère L1 à partir de leur mélange M1, avec une très bonne efficacité de collecte de la phase dense D1 et une distribution des temps de séjour de la phase légère L1, dans ledit séparateur, plus étroit que dans les séparateurs cycloniques de l'art antérieur. Le volume utile à la séparation pourra être, dans le séparateur cyclonique à co-courant particulier utilisé dans le procédé selon l'invention, plus faible que dans les cyclones de l'art antérieur, et par conséquent la séparation à débit de phase légère constant pourra être plus rapide.

Ce séparateur cyclonique à co-courant utilisé dans une forme préférée de réalisation de l'invention comporte en combinaison :

- au moins une enceinte extérieure, de forme allongée le long d'un axe, de section sensiblement circulaire de diamètre (Dc), comprenant à une première extrémité des moyens d'introduction permettant d'introduire, par une entrée dite entrée externe, le mélange M1 contenant les particules solides formant la phase dense D1 et les gaz formant la phase légère L1, lesdits moyens étant adaptés à conférer au moins à la phase légère L1 un mouvement hélicoïdal dans la direction de l'écoulement dudit mélange M1 dans ladite enceinte extérieure, comprenant également des moyens de séparation des phases D1 et L1 et à l'extrémité opposée à ladite première extrémité des moyens de récupération permettant de récupérer, par une sortie, comportant un conduit latéral ou axial, dite sortie externe, au moins une partie de la phase dense D1, et ayant entre lesdites extrémités opposées une longueur L,

- au moins une enceinte intérieure de forme allongée le long d'un axe, de section sensiblement circulaire, disposée coaxialement par rapport à ladite enceinte extérieure, comprenant à une distance Ls, inférieure à L, du niveau extrême de l'entrée externe, une entrée dite entrée interne, de diamètre intérieur (Di) inférieur à (Dc), dans laquelle pénètre au moins une partie de la phase légère L1 et à son extrémité opposée des moyens de récupération permettant de récupérer, par un conduit dit conduit interne, respectivement axial si le conduit de la sortie externe est latéral, ou latéral si le conduit de la sortie externe est axial, ladite partie de la phase légère L1,

- ledit séparateur cyclonique comportant en outre en aval, dans le sens de circulation de la phase dense D1, du niveau de l'entrée interne de l'enceinte intérieure, des moyens limitant la progression de la phase légère L1 à l'extérieur de ladite enceinte intérieure.

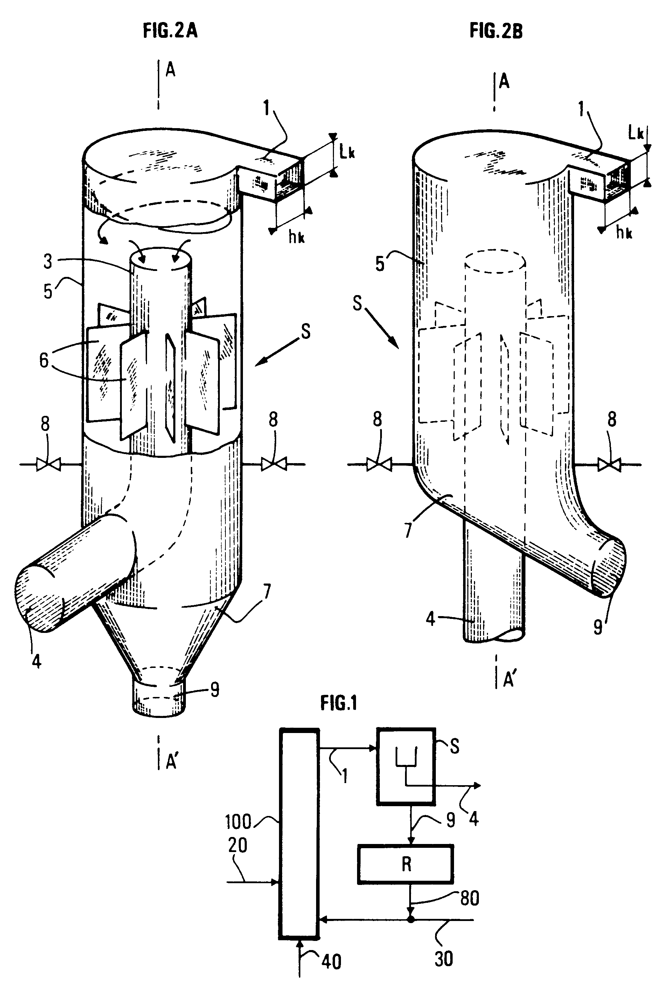

L'invention sera mieux comprise par la description de quelques modes de réalisation, donnés à titre purement illustratif mais nullement limitatif, qui en sera faite ci-après à l'aide des figures 1, 2A, 2B, 3, 4, 5 et 6 annexées, sur lesquelles les organes similaires sont désignés par les mêmes chiffres et lettres de référence.

Selon la figure 1, la conversion est effectuée dans un réacteur 100 dans lequel la charge est introduite par la conduite (20), le solide catalytique est introduit par le conduit (30) et le gaz d'entraînement du solide est introduit par le conduit (40) le réacteur est relié par un conduit (1) à un séparateur cyclonique (S) à co-courant permettant de séparer une phase solide d'une phase gazeuse, contenant les produits de la conversion, que l'on récupère par le conduit (4). La phase solide est envoyée, par le conduit (9), à un régénérateur (R) dans lequel les particules catalytiques sont au moins en partie régénérées avant d'être renvoyées, par le conduit (80) relié au conduit (30), dans le réacteur (100).

La figure 2A est une vue en perspective d'un séparateur cyclonique à co-courant utilisé dans une forme préférée selon l'invention et dénommé ci-après l'appareil.

La figure 2B est une vue en perspective d'un appareil utilisé dans le cadre de la présente invention qui ne diffère de celui représenté sur la figure 2A que par les moyens de récupération de la phase dense D1 et de la phase légère L1. Ces moyens permettent dans le cas de l'appareil schématisé sur la figure 2B une récupération par un conduit latéral de la phase dense D1 et une récupération par un conduit axial de la phase légère L1 et dans celui schématisé sur la figure 2A une récupération par un conduit axial de la phase dense D1 et une récupération par un conduit latéral de la phase légère L1.

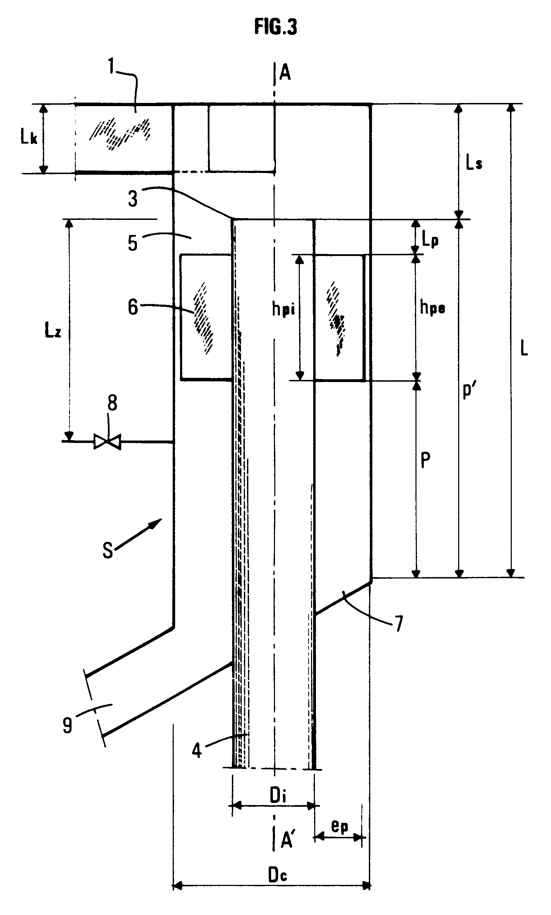

La figure 3 est une vue en coupe d'un appareil, utilisé dans le procédé selon l'invention, pratiquement identique à celui représenté sur la figure 2B mais comportant des moyens (6), limitant la progression de la phase légère L1 à l'extérieur de l'enceinte intérieure, dont la dimension dans la direction perpendiculaire à l'axe de l'enceinte extérieure est inférieure à la dimension de la sortie externe (5).

Les appareils, utilisés dans le cadre de l'invention, schématisés sur les figures 2A et 3, de formes allongées, sensiblement régulières, comportent une enceinte extérieure sensiblement verticale, ayant un axe (AA') qui est un axe de symétrie, de diamètre (Dc) et de longueur (L) entre le niveau extrême de l'entrée tangentielle (1), dite entrée externe, et les moyens (7) de sortie de la phase dense D1. Le mélange M1 contenant la phase dense D1 et la phase légère L1 est introduit par l'entrée tangentielle (1) suivant une direction sensiblement perpendiculaire à l'axe de l'enceinte extérieure. Cette entrée tangentielle a de préférence une section rectangulaire ou carrée dont le côté parallèle à l'axe de l'enceinte extérieure a une dimension (Lk) habituellement d'environ 0,25 à environ 1 fois le diamètre (Dc), et le côté perpendiculaire à l'axe de l'enceinte extérieure a une dimension (hk) habituellement d'environ 0,05 à environ 0,5 fois le diamètre (Dc).

Ces appareils comportent une enceinte intérieure, de forme allongée le long d'un axe, sensiblement verticale et de section sensiblement circulaire, disposée coaxialement par rapport à ladite enceinte extérieure, comprenant à une distance (Ls), inférieure à (L), du niveau extrême de l'entrée externe (1), une entrée (3) dite entrée interne, de diamètre (Di) inférieur à (Dc). Le diamètre de cette entrée interne (3) est habituellement d'environ 0,2 à environ 0,9 fois le diamètre (Dc), le plus souvent d'environ 0,4 à environ 0,8 fois le diamètre (Dc) et de préférence d'environ 0,4 à environ 0,6 fois le diamètre (Dc). Cette distance (Ls) est habituellement d'environ 0,2 à environ 9,5 fois le diamètre (Dc) et le plus souvent d'environ 0,5 à environ 2 fois le diamètre (Dc). Une distance relativement courte comprise entre 0,5 et 2 fois le diamètre (Dc) permet habituellement une séparation très rapide tout en conservant une bonne efficacité de séparation.

Les appareils comportent également en aval, dans le sens de circulation de la phase dense D1, du niveau de l'entrée interne (3), des moyens (6) limitant la progression de la phase légère L1 dans l'espace situé entre la paroi interne de l'enceinte extérieure et la paroi externe de l'enceinte intérieure ou sortie externe (5). Ces moyens (6) sont habituellement positionnés à l'intérieur de l'enceinte extérieure et l'extérieur de l'enceinte intérieure (entre la paroi externe de l'enceinte intérieure et la paroi interne de l'enceinte extérieure), entre le niveau de l'entrée interne (3) et les moyens (7) de récupération de la phase dense D1. Ces moyens (6) sont de préférence des pales sensiblement planes dont le plan passe par un axe sensiblement vertical et sont habituellement fixés sur au moins une paroi de l'une des enceintes intérieure ou extérieure. Ces moyens sont de préférence fixés à la paroi de l'enceinte intérieure de sorte que la distance (Lp) entre l'entrée interne et le point desdites pales le plus proche de cette entrée interne soit d'environ 0 à environ 5 fois le diamètre (Dc) et de préférence d'environ 0,1 à environ 1 fois ce diamètre (Dc).

Le nombre de pales est variable suivant la distribution du temps de séjour que l'on accepte pour la phase L1 et également en fonction du diamètre (Dc) de l'enceinte extérieure. Le nombre de pales est habituellement d'au moins 2 et par exemple de 2 à 50 et le plus souvent de 3 à 50. Les pales permettent une limitation de la continuation du vortex sur toute la section du cyclone, dans la sortie externe (5), autour du conduit formant l'enceinte intérieure et reliant l'entrée interne (3) à la sortie interne (4) de la phase légère, et donc une diminution et un contrôle de la distribution des temps de séjour de cette phase dans l'appareil.

Ainsi dans le cas de l'utilisation d'un appareil, tel que décrit ci-avant, dans le cadre du procédé selon l'invention on limite le temps de séjour de la phase légère L1 et la distribution de ces temps de séjour et en conséquence on limite ainsi la dégradation des produits contenus dans la phase légère circulant autour de l'entrée interne.

Chacune de ces pales a habituellement une dimension ou largeur (ep) mesurée dans la direction perpendiculaire à l'axe de l'enceinte intérieure (c'est-à-dire horizontalement, à partir de son arête la plus proche de l'axe de l'enceinte extérieure) et définie par rapport au diamètre intérieur (Dc) de l'enceinte extérieure et au diamètre extérieur (D'e) de l'enceinte intérieure d'environ 0,01 à 1 fois la valeur [((Dc)-(D'e))/2] de la demi différence de ces diamètres (Dc) et (D'e), de préférence d'environ 0,5 à environ 1 fois cette valeur et le plus souvent d'environ 0,9 à environ 1 fois cette valeur.

Dans le cas d'un appareil vertical, utilisé dans le procédé selon l'invention, tel que par exemple celui schématisé sur la figure 2A, ayant une sortie interne (4) latérale, et lorsque les pales sont positionnées après cette sortie interne, cette dimension (ep) peut être d'environ 0,01 à environ 1 fois la valeur (Dc)/2 du demi diamètre de l'enceinte extérieure.

Ces pales ont chacune sur leur arête, la plus proche de l'axe de l'enceinte intérieure, dans la direction paralléle à l'axe sensiblement vertical par lequel passe le plan de la pale, une dimension ou hauteur interne (hpi) et une dimension ou hauteur externe (hpe) mesurée dans la direction parallèle à l'axe sensiblement vertical par lequel passe le plan de la pale, sur l'arête de ladite pale la plus proche de la paroi interne de l'enceinte extérieure. Ces dimensions (hpi) et (hpe) sont habituellement supérieures à 0,1 fois le diamètre (Dc) et par exemple d'environ 0,1 fois à environ 10 fois le diamètre (Dc) et le plus souvent d'environ 1 à environ 4 fois ce diamètre (Dc). De préférence ces pales ont chacune une dimension (hpi) supérieure ou égale à leur dimension (hpe).

Selon la réalisation schématisée sur les figures 2A et 3 l'appareil comporte, en aval, dans le sens de l'écoulement des diverses phases, de l'entrée interne (3), au moins un moyen (8) permettant l'introduction éventuelle d'une phase légère L2 en un point situé entre l'entrée interne (3) de l'enceinte intérieure et l'extrémité du conduit (9) de récupération de la phase dense D1 ; ce ou ces points sont de préférence à une distance (Lz) de l'entrée (3) de l'enceinte intérieure. Ladite distance (Lz) a de préférence une valeur au moins égale à la somme des valeurs de (Lp) et (hpi) et au plus égale à la distance entre l'entrée (3) de l'enceinte intérieure et les moyens de sortie (7) de la phase dense D1. Cette phase légère L2 peut être introduite par exemple dans le cas où il est souhaitable d'effectuer un stripage de la phase dense D1. Dans ce cas cette phase légère constitue le fluide de stripage.

Cette phase légère L2 est de préférence introduite en plusieurs points qui sont habituellement répartis symétriquement, dans un plan au niveau duquel l'introduction est effectuée, autour de l'enceinte extérieure.

Le ou les points d'introduction de cette phase légère L2 sont habituellement situés à une distance au moins égale à 0,1 fois le diamètre (Dc) du point des dits moyens (6) le plus proche des moyens (7) de sortie de la phase dense D1. Le point d'introduction de cette phase légère L2 est de préférence situé à proximité du conduit (9) de récupération de la phase dense D1 et le plus souvent à proximité des moyens de sortie (7) de la phase dense D1.

La dimension (p') entre le niveau de l'entrée interne (3) et les moyens (7) de sortie de la phase dense D1 est déterminée à partir des autres dimensions des divers moyens formant l'appareil et de la longueur (L) de l'enceinte extérieure mesurée entre le niveau extrême de l'entrée tangentielle (1) et les moyens (7) de sortie de la phase dense D1. Cette dimension (L) est habituellement d'environ 1 à environ 35 fois le diamètre (Dc) de l'enceinte extérieure et le plus souvent d'environ 1 à 25 fois ce diamètre (Dc). On peut de même calculer la dimension (P), entre le point des moyens (6) le plus proche des moyens (7) de sortie de la phase dense D1 et lesdits moyens (7), à partir des autres dimensions des divers moyens formant l'appareil et de la longueur (L).

Les moyens (6) limitent la progression du vortex de la phase légère L1 dans la sortie externe (5). La position de ces moyens (6) et leur nombre influent donc sur les performances de la séparation des phases D1 et L1 contenues dans le mélange M1 (perte de charge et efficacité de la collecte des phases) et également sur la pénétration du vortex de la phase légère L1 dans la sortie (5). Ces paramètres seront donc choisis avec soin par l'homme du métier en particulier en fonction des résultats souhaités et de la perte de charge tolérée. En particulier le nombre de pales, leur forme et leur position seront choisis avec soins en tenant compte de leur influence sur l'écoulement du solide en liaison avec la limitation recherchée de la progression du vortex dans la sortie externe (5).

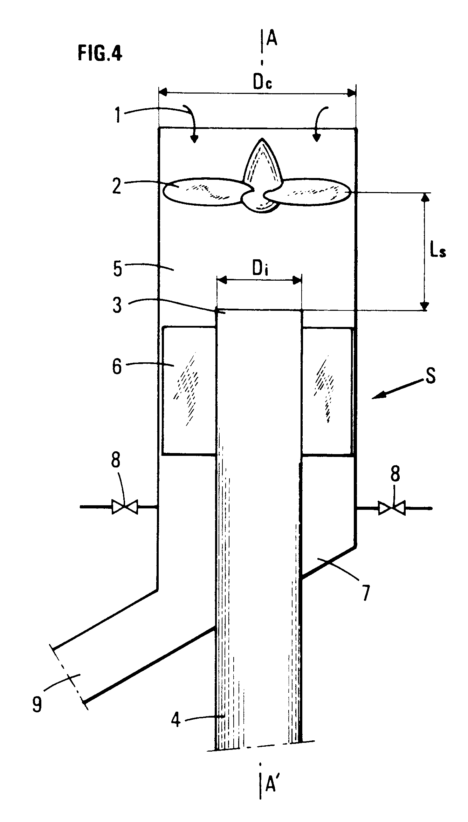

La figure 4 est une vue en perspective d'un appareil, utilisable dans le procédé selon l'invention, comportant une enceinte extérieure, de diamètre (Dc) ayant une entrée (1) dite entrée externe axiale, dans laquelle on introduit suivant une direction sensiblement parallèle à l'axe (AA') de l'enceinte extérieure le mélange M1 contenant la phase dense D1 et la phase légère L1. Cet appareil comporte en outre des moyens (2) placés à l'intérieur de l'entrée (1) permettant de conférer en aval, dans le sens de circulation dudit mélange M1, un mouvement hélicoïdal ou tourbillonnant au moins à la phase L1 dudit mélange M1. Ces moyens sont habituellement des pales inclinées. La longueur L de l'appareil est comptée entre ces moyens permettant de créer un vortex, au moins sur la phase L1, et les moyens (7) ce sortie de la phase dense D1. Toutes les autres caractéristiques sont identiques à celles décrites en liaison avec les appareils représentés sur les figures 2A et 3, en particulier les diverses dimensions sont celles mentionnées dans la description de ces appareils. Les variantes décritent en liaison avec les appareils représentés sur les figures 2A et 3 sont également possibles dans le cas de l'appareil schématisé sur la figure 4. On peut en particulier envisager une sortie interne (4) latérale et un conduit (9) de récupération de la phase dense D1 axial comme dans le cas de la réalisation schématisée sur la figure 2B.

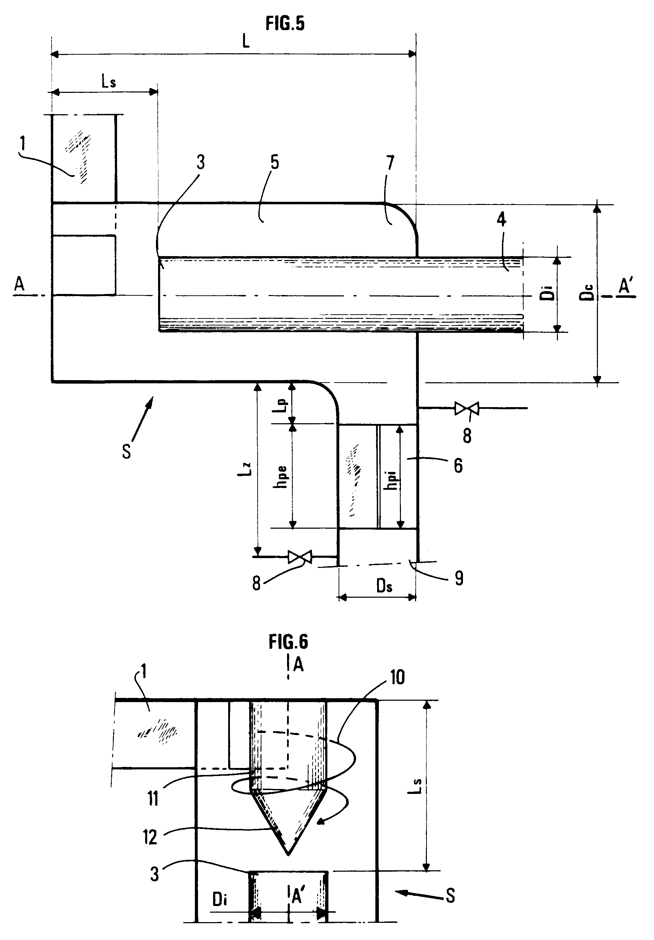

La figure 5 est une vue en coupe d'un appareil, utilisable dans le procédé selon l'invention, de forme allongée, sensiblement régulière, comportant une enceinte extérieure, ayant un axe (AA') qui est un axe de symétrie, sensiblement horizontale de diamètre (Dc) et de longueur (L) entre entre le niveau extrême de l'entrée tangentielle (1), dite entrée externe, et les moyens (7) de sortie de la phase dense D1. Le mélange M1 contenant la phase dense D1 et la phase légère L1 est introduit par l'entrée tangentielle (1) suivant une direction sensiblement perpendiculaire à l'axe de l'enceinte extérieure.

Cet appareil comporte également en aval, dans le sens de circulation de la phase dense D1, du niveau de l'entrée interne (3), des moyens (6) limitant la progression de la phase légère L1, à l'extérieur de l'enceinte intérieure, dans l'espace situé entre la paroi interne de l'enceinte extérieure et la paroi externe de l'enceinte intérieure ou sortie externe (5). Ces moyens (6) sont habituellement positionnés, en aval, dans le sens de circulation de la phase dense D1, des moyens de récupération (7) de la phase dense D1, dans le conduit (9), de récupération de la phase dense D1, de diamètre (Ds).

Ces moyens (6) sont habituellement des pales sensiblement planes dont le plan passe par un axe sensiblement vertical. La dimension (ep) de chacune de ces pales est habituellement d'environ 0,01 à environ 1 fois le diamètre (Ds) du conduit (9). Les pales sont habituellement positionnées de manière à ce que l'arête intérieure, c'est-à-dire l'arête de la pale la plus proche de l'axe du conduit (9), de chacune d'elles soit confondue avec l'axe dudit conduit (9). Ces pales sont positionnées à une distance (Lp) par rapport aux moyens de séparation (7) d'environ 0 à environ 5x(Dc).

Les moyens (8) permettant d'introduire éventuellement une phase légère L2 sont habituellement positionnés en aval, dans le sens de circulation de la phase dense D1, du niveau de l'entrée interne (3), et de préférence entre les moyens (7) de récupération de la phase dense D1 et l'extrémité du conduit (9) de récupération de la phase dense D1. Dans le cas de l'appareil schématisé sur la figure 5 l'introduction d'une phase légère L2 est prévue à 2 niveaux différents par un premier moyen (8) au niveau des moyens (7) et par un deuxième moyen (8) en dessous des moyens (6). Les moyens (8) sont positionnés à une distance (Lz), des moyens de récupération de la phase dense D1, mesurée à partir desdits moyens (7).

Cet appareil schématisé sur la figure 5 comporte un conduit (9), de récupération de la phase dense D1, de diamètre (Ds) habituellement égal à environ 0,1 à environ 1 fois le diamètre (Dc) et le plus souvent d'environ 0,2 à environ 0,7 fois ce diamètre.

Toutes les autres caractéristiques de ce séparateur cyclonique horizontal sont identiques à celles décrites en liaison avec les appareils représentés sur les figures 2A et 3, en particulier les diverses dimensions sont celles mentionnées dans la description de ces appareils.

Bien que cela ne soit pas réprésenté sur les figures 2A, 2B, 3, 4 et 5 il est possible, et habituellement souhaitable, dans le cas de débits importants des diverses phases au niveau des entrées de l'appareil, d'utiliser des moyens permettant de favoriser la formation du vortex. De tel moyens (10) sont par exemple représentés sur la figure 6 qui représente selon une réalisation préférée de l'appareil, utilisé dans le procédé selon l'invention, la partie voisine de l'entrée tangentielle (1) du mélange M1. Selon cette réalisation l'appareil comporte un toit (10), par exemple hélicoïdal, descendant à partir du niveau extrême de l'entrée tangentielle (1). Ces moyens (10) peuvent également consister en une volute interne ou externe. Ces moyens permettent en outre de limiter les interférences entre le flux du mélange M1 et les flux des phases déjà présentes dans le séparateur et de limiter également la turbulence au niveau de l'entrée tangentielle (1). Habituellement, en particulier dans le cas d'un toit hélicoïdal descendant, le pas de l'hélice est d'environ 0,01 à environ 3 fois la valeur de (Lk) et le plus souvent d'environ 0,5 à environ 1,5 fois cette valeur.

Dans cette forme préférée de réalisation de l'appareil, utilisé dans le cadre du procédé de l'invention, celui-ci comporte également entre l'entrée externe et l'entrée interne des moyens de stabilisation de l'écoulement hélicoïdal d'au moins la phase légère L1 et de limitation du volume utile à la séparation. Ces moyens sont de préférence centrés sur l'axe de l'enceinte intérieure.

Ces moyens peuvent être un cône dont la pointe est dirigée vers l'entrée interne et dont la base est située au niveau extrême de l'entrée tangentielle (1). Ils peuvent aussi être formés, comme cela est schématisé sur la figure 6, par un cylindre (11) prolongé par un cône (12). Le diamètre de la base du cône est identique à celui du cylindre et est strictement inférieur au diamètre (Dc). Ce diamètre est habituellement d'environ 0,01 à environ 1,5 fois le diamètre (Di) de l'entrée interne (3) et de préférence d'environ 0,75 à environ 1,25 fois le diamètre (Di). L'encombrement axial ou dimension entre le niveau extrême de ces moyens le plus proche de l'entrée tangentielle et l'extrémité opposée desdits moyens est habituellement d'environ 0,01 à environ 3 fois la valeur (Ls) de la distance entre le niveau extrême de l'entrée tangentielle (1) et le niveau de l'entrée interne (3) et de préférence d'environ 0,75 à environ 1,25 fois cette valeur (Ls).

Les moyens de sortie (7) de la phase dense D1 permettent habituellement de collecter et de canaliser cette phase dense D1 jusqu'à la sortie externe (9). Ces moyens sont le plus souvent un fond incliné ou un cône axé ou non sur la sortie interne (4).

Les appareils, décrits ci-avant et utilisés dans le cadre du procédé selon la présente invention, permettent ainsi la séparation rapide, à partir d'un mélange M1, comprenant une phase dense et une phase légère, de ladite phase dense et de ladite phase légère.

Le diamètre (Dc) de l'appareil mesuré au niveau de l'entrée tangentielle (1) du côté de son extrémité la plus proche de l'entrée interne (3) est habituellement d'environ 0,01 à environ 10 m (mètres) et le plus souvent d'environ 0,05 à environ 2 m. Il est habituellement préférable de garder un diamètre constant sur toute la longueur de l'appareil comprise entre l'extrémité de l'entrée tangentielle la plus proche de l'entrée interne (3) et ladite entrée interne (3) ou même depuis le niveau de l'injection du mélange M1 jusqu'au niveau des moyens (7) de sortie de la phase dense D1 ; cependant on peut envisager la réalisation et l'utilisation d'un appareil comportant des élargissements ou des rétrécissements de section entre lesdits niveaux.

Pour obtenir une bonne séparation de la phase légère L1 contenu dans le mélange M1 comprenant également la phase dense D1 il est préférable d'avoir une vitesse superficielle d'entrée de cette phase L1 élevée. Cette vitesse sera par exemple d'environ 0,1 à environ 250 mxs-1 (mètre par seconde), de préférence d'environ 0,5 à environ 75 mxs-1 et le plus souvent d'environ 1 à 20 mxs-1. Le rapport en poids du débit de la phase D1 au débit de la phase L1 est habituellement d'environ 2 : 1 à environ 100 : 1 et le plus souvent d'environ 5 : 1 à environ 50 : 1.

Il est possible en augmentant la différence de pression entre l'entrée (3) et les moyens (7), ce qui peut être obtenu par exemple en augmentant la pression en aval, dans le sens de la circulation de la phase dense D1, de l'entrée interne (3) ou en diminuant la pression en aval, dans le sens de la circulation de la phase dense D1, des moyens (7) de sortie de cette phase, de soutirer une partie plus ou moins importante de la phase L1 avec la phase D1 et simultanément d'obtenir au niveau de la sortie (4) un mélange pratiquement complètement exempt de phase D1. On peut ainsi soutirer par exemple environ 1 à environ 10 % de la phase L1 avec D1. Les variations de pression permettant de jouer sur la quantité de phase L1 soutirée avec la phase D1 sont assurées par des moyens bien connus de l'homme du métier et par exemple en modifiant le débit de la phase L2, ou en modifiant les conditions d'opérations en aval de la sortie (9). Ainsi dans une forme particulière de réalisation de l'invention l'appareil comprendra au moins un moyen permettant le soutirage, par la sortie externe (5), d'au moins une partie de la phase légère L1 en mélange avec la phase dense D1.

Dans les divers appareils utilisés dans le procédé selon l'invention et dans les différents modes d'injection du mélange M1 un tel soutirage peut permettre d'améliorer l'efficacité de récupération de la phase dense D1.

Le choix entre un appareil comportant une entrée tangentielle, pour le mélange M1, et un appareil comportant une entrée axiale, pour ce mélange M1, est habituellement guidé par le rapport en poids des débits des phases L1 et D1. Dans le cas où ce rapport est inférieur à 5 : 1, il peut être avantageux de choisir un appareil à entrée axiale.

La présente invention concerne également un dispositif pour la conversion catalytique en lit entraîné d'une charge contenant au moins un composé oxygéné, en hydrocarbures oléfiniques riches en composés ayant de 2 à 4 atomes de carbone dans leur molécule, comportant une enceinte de forme allongée, dans laquelle ladite conversion est effectuée dans des conditions appropriées, comprenant, à proximité d'une première extrémité, d'amont en aval dans le sens du déplacement de la charge, au moins un moyen d'introduction d'au moins un fluide de d'entraînement, au moins un moyen d'introduction d'au moins un solide contenant des particules catalytiques, au moins un moyen d'introduction de ladite charge, ledit dispositif comprenant à proximité d'une deuxième extrémité de ladite enceinte au moins un moyen, relié à la dite enceinte, de séparation des particules solides et des gaz contenant les produits de la conversion, au moins partielle, de ladite charge et au moins un moyen de régénération d'au moins une partie des particules solides catalytiques relié d'une part au moyen de séparation desdites particules solides et d'autre part à ladite enceinte à proximité de ladite première extrémité de manière à permettre la régénération d'au moins une partie des particules solides catalytiques et le recyclage desdites particules solides dans ladite enceinte caractérisé en ce que ledit moyen de séparation des particules solides et des gaz est un séparateur cyclonique à co-courant. Ledit dispositif comprend de préférence un séparateur cyclonique à co-courant comportant au moins un moyen d'introduction d'au moins un fluide de stripage.

Le séparateur cyclonique à co-courant que l'on utilise de préférence comporte en combinaison :

- au moins une enceinte extérieure, de forme allongée le long d'un axe, de section sensiblement circulaire de diamètre (Dc), comprenant à une première extrémité des moyens d'introduction permettant d'introduire, par une entrée dite entrée externe, le mélange M1 contenant les particules solides formant la phase dense D1 et les gaz formant la phase légère L1, lesdits moyens étant adaptés à conférer au moins à la phase légère L1 un mouvement hélicoïdal dans la direction de l'écoulement dudit mélange M1 dans ladite enceinte extérieure, comprenant également des moyens de séparation des phases D1 et L1 et à l'extrémité opposée à ladite première extrémité des moyens de récupération permettant de récupérer, par une sortie, comportant un conduit latéral ou axial, dite sortie externe, au moins une partie de la phase dense D1, et ayant entre lesdites extrémités opposées une longueur L,

- au moins une enceinte intérieure de forme allongée le long d'un axe, de section sensiblement circulaire, disposée coaxialement par rapport à ladite enceinte extérieure, comprenant à une distance Ls, inférieure à L, du niveau extrême de l'entrée externe, une entrée dite entrée interne, de diamètre (Di) inférieur à (Dc), dans laquelle pénètre au moins une partie de la phase légère L1 et à son extrémité opposée des moyens de récupération permettant de récupérer, par un conduit dit conduit interne, respectivement axial si le conduit de la sortie externe est latéral, ou latéral si le conduit de la sortie externe est axial, ladite partie de la phase légère L1,

- ledit séparateur cyclonique comportant en outre en aval, dans le sens de circulation de la phase dense D1, du niveau de l'entrée interne de l'enceinte intérieure, des moyens limitant la progression de la phase légère L1 à l'extérieur de ladite enceinte intérieure.

Dans le cadre de la présente invention il n'est pas fait mention des solides et catalyseurs employés, ni des divers fluides utilisés qui sont des données classiques bien connues de l'homme de la conversion catalytique des composés oxygénés en hydrocarbures oléfiniques. Le fluide d'entraînement est le plus souvent choisi dans la groupe formé par la vapeur d'eau, les gaz inertes ou les hydrocarbures gazeux ou des mélanges de ces composés. Les conditions de mise en oeuvre de la réaction sont souvent telles que l'on opère en lit fluidisé entraîné. Dans ce cas le fluide de fluidisation est le plus souvent le même que le fluide d'entraînement. Ce fluide unique est alors divisé en deux fractions l'une formant le fluide de fluidisation et l'autre le fluide d'entraînement.

Les catalyseurs employés sont de préférence des catalyseurs zéolithiques.

Les conditions opératoires relatives aux procédés de conversion de charges oxygénées comme le méthanol sont par exemples décrites dans les brevets suivants : US-A-4689205, US-A-3969426 et US-A-4229608, mais aucun de ces brevets ne décrit l'utilisation d'un séparateur cyclonique à co-courant pour améliorer la rapidité de la séparation solide-gaz.

Les conditions opératoires de la conversion de la charge oxygénée sont en général les suivantes :

- la charge, qui le plus souvent contient un alcool et habituellement le méthanol, renferme généralement de 50 à 100 %, de préférence de 70 à 90 % en poids de composés oxygénés et de 0 à 50 %, de préférence de 5 à 30 % en poids d'eau. La charge peut également contenir un gaz inerte tel que par exemple l'azote seul ou en mélange avec la vapeur d'eau dans la proportion indiquée ci-avant pour la vapeur d'eau. Dans le cas le plus fréquent où la charge contient du méthanol elle peut être constituée par du méthanol brut tel que par exemple du méthanol provenant d'une unité de synthèse de ce composé à partir d'un gaz contenant du monoxyde de carbone,

- la température d'injection est habituellement égale à la température de rosée de la charge,

- la pression absolue régnant dans l'unité de conversion est habituellement de 0,1 à 0,5 mégapascal (MPa) et le plus souvent d'environ 0,2 MPa,

- la température de la zone réactionnelle est habituellement de 500 à 620 °C et le plus souvent de 550 à 590 °C,

- le temps de séjour dans la zone réactionnelle est habituellement de 0,05 à 10 secondes et le plus souvent de 0,5 à 3 secondes,

- la vitesse des gaz dans la zone réactionnelle est habituellement de 0,5 à 40 m/s et le plus souvent de 1 à 20 m/s,

- la vitesse des solides dans la zone réactionnelle est habituellement voisine de celle des gaz et généralement de 0,5 à 40 m/s et le plus souvent de 1 à 20 m/s.

Les conditions opératoires sont choisies pour que la composition des produits obtenus exprimée par le rendement en carbone en composés éthyléniques ayant de 2 à 4 atomes de carbone dans leur molécule soit supérieur à 70 % et de préférence supérieur à 80 %. Les produits obtenus peuvent être particulièrement riches en éthylène (rendement en carbone pour l'éthylène supérieur à 40 %) ou particulièrement riches en propylène (rendement en carbone pour le propylène supérieur à 60 %).

La masse volumique des solides est habituellemnt de 10 à 700 kilogramme par mètre cube (kg/m3) et le plus souvent de 20 à 400 kg/m3.

Le rapport C/O du débit massique de catalyseur au débit massique de charge est habituellement d'environ 2 : 1 à environ 50 : 1 et le plus souvent d'environ 5 : 1 à environ 30 : 1.

## Revendications

1. Procédé de conversion catalytique d'une charge comprenant au moins un composé oxygéné, en hydrocarbures oléfiniques riches en composés ayant de 2 à 4 atomes de carbone dans leur molécule comprenant la conversion en lit entraîné de ladite charge, dans une zone réactionnelle de conversion de forme allongée, dans des conditions appropriées, en présence d'un catalyseur sous forme de particules solides, ledit procédé comportant une étape d'alimentation, dans une zone située à proximité d'une première extrémité de ladite zone réactionnelle, en au moins un solide sous forme de particules contenant des particules catalytiques et en au moins un fluide d'entraînement, une étape d'introduction, et, dans le cas d'une charge au moins en partie liquide, de pulvérisation de ladite charge dans une zone d'introduction située en aval, dans le sens du déplacement des particules solides, de la zone d'introduction desdites particules solides, une étape de mise en contact desdites particules solides et de ladite charge dans une zone située à proximité de la première extrémité, une étape de circulation des particules solides et de la charge dans la zone de réaction au cours de laquelle on effectue la conversion de ladite charge et on désactive au moins en partie les particules solides catalytiques par dépôt de coke sur celles-ci, une étape de séparation d'un mélange M1 de particules solides et de gaz contenant les produits de la conversion, au moins partielle, de ladite charge, dans une zone de séparation située à proximité d'une deuxième extrémité de la zone de réaction à l'opposé de ladite première extrémité, une étape de régénération, dans au moins une zone de régénération, d'au moins une partie des particules solides catalytiques, au moins en partie désactivées et une étape de recyclage des particules solides catalytiques, au moins en partie régénérées, dans une zone de recyclage à proximité de ladite première extrémité caractérisé en ce que la séparation des particules solides et des gaz contenant les produits de la réaction est effectuée dans un séparateur cyclonique à co-courant.

2. Procédé selon la revendication 1 dans lequel le séparateur cyclonique à co-courant comporte en combinaison :
   - au moins une enceinte extérieure, de forme allongée le long d'un axe, de section sensiblement circulaire de diamètre (Dc), comprenant à une première extrémité des moyens d'introduction permettant d'introduire, par une entrée dite entrée externe, le mélange M1 contenant les particules solides formant une phase dense D1 et les gaz formant une phase légère L1, lesdits moyens étant adaptés à conférer au moins à la phase légère L1 un mouvement hélicoïdal dans la direction de l'écoulement dudit mélange M1 dans ladite enceinte extérieure, comprenant également des moyens de séparation des phases D1 et L1 et à l'extrémité opposée à ladite première extrémité des moyens de récupération permettant de récupérer, par une sortie, comportant un conduit latéral ou axial, dite sortie externe, au moins une partie de la phase dense D1, et ayant entre lesdites extrémités opposées une longueur L,
   - au moins une enceinte intérieure de forme allongée le long d'un axe, de section sensiblement circulaire, disposée coaxialement par rapport à ladite enceinte extérieure, comprenant à une distance Ls, inférieure à L, du niveau extrême de l'entrée externe, une entrée dite entrée interne, de diamètre (Di) inférieur à (Dc), dans laquelle pénètre au moins une partie de la phase légère L1 et à son extrémité opposée des moyens de récupération permettant de récupérer, par un conduit dit conduit interne, respectivement axial si le conduit de la sortie externe est latéral, ou latéral si le conduit de la sortie externe est axial, ladite partie de la phase légère L1,
   - ledit séparateur cyclonique comportant en outre en aval, dans le sens de circulation de la phase dense D1, du niveau de l'entrée interne de l'enceinte intérieure, des moyens limitant la progression de la phase légère L1 à l'extérieur de ladite enceinte intérieure.

3. Procédé selon la revendication 1 ou 2 dans lequel le séparateur cyclonique à co-courant comporte au moins un moyen d'introduction d'au moins un fluide permettant d'effectuer le strippage des particules solides.

4. Procédé selon la revendication 3 dans lequel l'enceinte extérieure est sensiblement verticale et les moyens limitant la progression de la phase légère L1 à l'extérieur de l'enceinte intérieure sont positionnés à l'intérieur de l'enceinte extérieure et à l'extérieur de l'enceinte intérieure, entre le niveau de l'entrée interne et les moyens de récupération de la phase dense D1.

**5.** Procédé selon la revendication 3 dans lequel l'enceinte extérieure est sensiblement horizontale et les moyens limitant la progression de la phase légère L1 à l'extérieur de l'enceinte intérieure sont positionnés, en aval, dans le sens de circulation de la phase dense D1, des moyens de récupération de la phase dense D1, dans le conduit de la sortie externe.

**6.** Procédé selon l'une des revendications 3 à 5 dans lequel les moyens limitant la progression de la phase légère L1 à l'extérieur de l'enceinte intérieure sont des pales sensiblement planes dont le plan passe par un axe sensiblement vertical.

**7.** Procédé selon la revendication 6 dans lequel le séparateur cyclonique comporte de 2 à 50 pales ayant chacune une dimension (ep), mesurée, horizontalement, à partir de son arête la plus proche de l'axe de l'enceinte extérieure d'environ 0,01 à environ 1 fois la valeur [((Dc)-(D'e))/2] lorsque ces pales sont, dans le cas d'un séparateur cyclonique vertical, positionnées entre la paroi externe de l'enceinte intérieure de diamètre externe (D'e) et la paroi interne de l'enceinte extérieure de diamètre intérieur (Dc), d'environ 0,01 à environ 1 fois la valeur (Dc)/2 dans le cas d'un séparateur cyclonique vertical à sortie interne latérale lorsqu'elles sont positionnées après cette sortie interne et d'environ 0,01 à 1 fois le diamètre (Ds) du conduit de la sortie externe dans le cas d'un séparateur cylonique horizontal, une dimension (hpe), mesurée, dans la direction parallèle à l'axe sensiblement vertical par lequel passe le plan de la pale, sur l'arête de la pale la plus proche de la paroi interne de l'enceinte extérieure ou de la paroi interne de la sortie externe et une dimension (hpi) mesurée, sur l'arête de la pale la plus proche de l'axe de l'enceinte intérieure ou de l'axe de la sortie externe, dans la direction parallèle à l'axe sensiblement vertical par lequel passe le plan de la pale, lesdites dimensions (hpe) et (hpi) étant d'environ 0,1x(Dc) à environ 10x(Dc) et lesdites pales étant chacune situées à une distance, par rapport à l'entrée interne dans le cas d'un séparateur cyclonique vertical ou par rapport aux moyens de séparation dans le cas d'un séparateur cyclonique horizontal, d'environ 0 à environ 5x(Dc).

**8.** Procédé selon la revendication 7 dans lequel les pales ont chacune une dimension (hpi) supérieure ou égale à (hpe).

**9.** Procédé selon l'une des revendications 2 à 8 dans lequel le moyen d'introduction d'au moins un fluide constituant une phase légére L2 permettant d'effectuer simultanément le strippage des particules solides est positionné entre l'entrée interne de l'enceinte intérieure et l'extrémité du conduit de la sortie externe du séparateur cyclonique.

**10.** Procédé selon l'une des revendications 3 à 9 dans lequel le séparateur cyclonique comprend entre l'entrée externe et l'entrée interne des moyens de stabilisation de l'écoulement hélicoïdal d'au moins la phase légère L1 et de limitation du volume utile à la séparation.

**11.** Procédé selon l'une des revendications 3 à 10 dans lequel le mélange M1 est introduit selon une direction sensiblement parallèle à l'axe de l'enceinte extérieure ou dans une direction sensiblement perpendiculaire à l'axe de l'enceinte extérieure.

**12.** Procédé selon l'une des revendications 3 à 11 dans lequel le séparateur cyclonique comprend des moyens, de limitation des interférences entre le flux du mélange M1 introduit et les flux des phases déjà présentes dans le séparateur, choisis parmi un toit descendant, une volute externe et une volute interne.

**13.** Procédé selon l'une des revendications 1 à 12 dans lequel la charge comprend au moins un alcool, de préférence le méthanol et dans lequel elle est introduite à l'état gazeux.

**14.** Dispositif de conversion catalytique en lit entraîné d'une charge contenant au moins un composé oxygéné, en hydrocarbures oléfiniques riches en composés ayant de 2 à 4 atomes de carbone dans leur molécule, comportant une enceinte de forme allongée, dans laquelle ladite conversion est effectuée dans des conditions appropriées, comprenant, à proximité d'une première extrémité, d'amont en aval dans le sens du déplacement de la charge, au moins un moyen d'introduction d'au moins un fluide d'entraînement, au moins un moyen d'introduction d'au moins un solide contenant des particules catalytiques, au moins un moyen d'introduction de ladite charge, ledit dispositif comprenant à proximité d'une deuxième extrémité de ladite enceinte au moins un moyen, relié à la dite enceinte, de séparation des particules solides et des gaz contenant les produits de la conversion, au moins partielle, de ladite charge et au moins un moyen de régénération d'au moins une partie des particules solides catalytiques relié d'une part au moyen de séparation desdites particules solides et d'autre part à ladite enceinte à proximité de ladite première extrémité de manière à permettre la régénération d'au moins une partie des particules solides catalytiques et le recyclage desdites particules solides dans ladite enceinte caractérisé en ce que ledit moyen de séparation des particules solides et des gaz est un séparateur cyclonique à co-courant.

**15.** Dispositif selon la revendication 14 dans lequel le séparateur cyclonique à co-courant comporte en combinaison :

- au moins une enceinte extérieure, de forme allongée le long d'un axe, de section sensiblement circulaire de diamètre (Dc), comprenant à une première extrémité des moyens d'introduction permettant d'introduire, par une entrée dite entrée externe, le mélange M1 contenant les particules solides formant la phase dense D1 et les gaz formant la phase légère L1, lesdits moyens étant adaptés à conférer au moins à la phase légère L1 un mouvement hélicoïdal dans la direction de l'écoulement dudit mélange M1 dans ladite enceinte extérieure, comprenant également des moyens de séparation des phases D1 et L1 et à l'extrémité opposée à ladite première extrémité des moyens de récupération permettant de récupérer, par une sortie, comportant un conduit latéral ou axial, dite sortie externe, au moins une partie de la phase dense D1, et ayant entre lesdites extrémités opposées une longueur L,
- au moins une enceinte intérieure de forme allongée le long d'un axe, de section sensiblement circulaire, disposée coaxialement par rapport à ladite enceinte extérieure, comprenant à une distance Ls, inférieure à L, du niveau extrême de l'entrée externe, une entrée dite entrée interne, de diamètre (Di) inférieur à (Dc), dans laquelle pénètre au moins une partie de la phase légère L1 et à son extrémité opposée des moyens de récupération permettant de récupérer, par un conduit dit conduit interne, respectivement axial si le conduit de la sortie externe est latéral, ou latéral si le conduit de la sortie externe est axial, ladite partie de la phase légère L1,
- ledit séparateur cyclonique comportant en outre en aval, dans le sens de circulation de la phase dense D1, du niveau de l'entrée interne de l'enceinte intérieure, des moyens limitant la progression de la phase légère L1 à l'extérieur de ladite enceinte intérieure.

## Claims

**1.** Process for the catalytic conversion of a charge containing at least one oxygen compound into olefinic hydrocarbons rich in compounds having 2 to 4 carbon atoms in their molecule, comprising the entrained bed conversion of said charge, in an elongated conversion reaction zone, under appropriate conditions and in the presence of a catalyst in the form of solid particles, said process having a stage of supplying, to a zone located in the vicinity of a first end of said reaction zone, at least one solid in the form of particles containing catalytic particles and at least one entrainment fluid, an introduction stage and, in the case of an at least partly liquid charge the spraying of said charge into an introduction zone located downstream in the displacement direction of the solid particles from the introduction zone of said solid particles, a stage of contacting said solid particles and said charge in a zone located in the vicinity of the first end, a stage of circulating the solid particles and the charge into the reaction zone and during which said charge is converted and the at least partial deactivation of the catalytic solid particles by the deposition of coke thereof, a stage of at least partial separation a mixture M1 containing the products of the at least partial conversion of said charge in a separation zone located in the vicinity of a second end of the reaction zone opposite to said first end, a regeneration stage, in at least one regeneration zone, of at least one part of the solid catalytic particles, which are at least partly deactivated and a stage of recycling the solid catalytic particles, which are at least partly regenerated, in a recycling zone in the vicinity of said first end, characterized in that the separation of the solid particles and the gases containing the products of the reaction is performed in a co-current cyclone separator.

**2.** Process according to claim 1, wherein the co-currant cyclone separator has in combination:

- at least one external enclosure, elongated along an axis, having a substantially circular section of diameter (Dc) and having at a first end introduction means making it possible to introduce by an external inlet the mixture M1 containing the solid particles forming the dense phase D1 and the gases forming the light phase L1, said means being appropriate to give at least to the said light phase L1 a helical movement in the flow direction of said mixture M1 into said external enclosure, whilst also comprising means for separating the phases D1 and L1 and at the end opposite to said first end recovery means making it possible to recover by an outlet having a lateral or axial pipe and referred to as the external outlet, at least part of the dense phase D1 and having between said opposite ends a length L,

- at least one internal enclosure elongated along an axis and having a substantially circular section arranged coaxially with respect to said external enclosure and having at a distance Ls, smaller than L, from the extreme level of the external inlet, a so-called internal inlet having an internal diameter (Di) smaller than (Dc) and which is penetrated by at least part of the light phase L1 and at its opposite end recovery means making it possible to recover by a so-called internal pipe, which is respectively axial if the pipe of the external outlet is lateral, or lateral if the pipe of the external outlet is axial, said part of the light phase L1,

- said cyclone separator also having downstream, in the circulation direction of the dense phase D1 from the level of the internal inlet of the internal enclosure, means limiting the advance of the light phase L1 outside said internal enclosure.

3. Process according to claims 1 or 2, wherein the co-current cyclone separator has at least one means for introducing at least one fluid making it possible to strip the solid particles.

4. Process according to claim 3, wherein the external enclosure is substantially vertical and the means limiting the advance of the light phase L1 to the outside of the internal enclosure are positioned within the external enclosure and outside the internal enclosure, between the level of the internal inlet and the recovery means for the dense phase D1.

5. Process according to claim 3, wherein the external enclosure is substantially horizontal and the means limiting the advance of the light phase L1 to the outside of the internal enclosure are positioned, downstream, in the flow direction of the dense phase D1, of the recovery means for said dense phase D1 in the external outlet pipe.

6. Process according to any one of the claims 3 to 5, wherein the means limiting the advance of the light phase L1 to the exterior of the internal enclosure are substantially planar blades, whose plane passes through a substantially vertical axis.

7. Process according to claim 6, wherein the cyclone separator has 2 to 50 blades each having a dimension (ep), measured horizontally, from its edge closest to the axis of the external enclosure approximately 0.01 to approximately once the value $[((Dc)-(De))/2]$ when these blades are, in the case of a vertical cyclone separator, positioned between the outer wall of the internal enclosure of external diameter (De) and the inner wall of the external enclosure of internal diameter (Dc), of approximately 0.01 to approximately once the value (Dc)/2 in the case of a vertical cyclone separator at the lateral internal outlet, when they are positioned after said internal outlet and approximately 0.01 to once the diameter (Ds) of the external discharge pipe in the case of a horizontal cyclone separator, a dimension (hpe), measured in the direction parallel to the substantially vertical axis through which passes the plane of the blade, on the edge of the blade closest to the inner wall of the external enclosure of the inner wall of the external outlet and a dimension (hpi) measured, on the edge of the blade closest to the axis of the internal enclosure or the axis of the external outlet, in the direction parallel to the substantially vertical axis through which passes the plane of the blade, said dimensions (hpe) and (hpi) being approximately 0.1x(Dc) to approximately 10x(Dc) and said blades being in each case located at a distance, relative to the internal inlet in the case of a vertical cyclone separator, or relative to the separating means in the case of a horizontal cyclone separator, of approximately 0 to approximately 5x(Dc).

8. Process according to claim 7, wherein each of the blades has a dimension (hpi) equal to or exceeding (hpe).

9. Process according to any one of the claims 2 to 8, wherein the introduction means of at least one fluid constituting a light phase L2 making it possible to carry out simultaneously the stripping of the solid particles is positioned between the internal inlet of the internal enclosure and the end of the external discharge pipe of the cyclone separator.

10. Process according to any one of the claims 3 to 9, wherein the cyclone separator has between the external inlet and the internal inlet, means for stabilizing the helical flow of at least the light phase L1 and for limiting the separation-useful volume.

11. Process according to any one of the claims 3 to 10, wherein the mixture M1 is introduced in a direction substantially parallel to the axis of the external enclosure or in a direction substantially perpendicular to the axis of the external enclosure.

12. Process according to any one of the claims 3 to 11, wherein the cyclone separator comprises means for limiting interference between the flow of the mixture M1 introduced and the flow of the phases already present in the separator and chosen from among a descending top, an external helix and an internal helix.

13. Process according to any one of the claims 1 to 12, wherein the charge comprises at least one alcohol, preferably methanol and wherein it is introduced in the gaseous phase.

14. Apparatus for the entrained bed catalytic conversion of a charge containing at least one oxygen compound into olefinic hydrocarbons rich in compounds having 2 to 4 carbon atoms in their molecule, having an elongated enclosure in which said conversion takes place under appropriate conditions and having, in the vicinity of a first end, from upstream to downstream in the displacement direction of the charge, at least one means for introducing at least one entrainment fluid, at least one means for introducing at least one solid containing catalytic particles, at least one means for introducing said charge, the apparatus having in the vicinity of a second end of said enclosure at least one means, connected to the said enclosure, for separating solid particles and gases containing the products of the at least partial conversion of said charge and at least one means for regenerating at least part of the solid catalytic particles connected on the one hand to the separation means for said solid particles and on the other to the said enclosure in the vicinity of said first end, so as to permit the regeneration of at least part of the solid catalytic particles and the recycling of said solid particles into said enclosure, characterized in that said means for separating the solid particles and the gases is a co-current cyclone separator.

15. Apparatus according to claim 14, wherein the co-current cyclone separator comprises in combination:
   - at least one external enclosure, elongated along an axis and having a substantially circular cross-section of diameter (Dc), having at a first end introduction means making it possible to introduce, by a so-called external inlet, the mixture M1 containing the solid particles forming the dense phase D1 and the gases forming the light phase L1, said means being able to give at least to the light phase L1 a helical movement in the flow direction of said mixture M1 into said external enclosure, also having means for separating the phases D1 and L1 and at the end opposite to said first end recovery means permitting the recovery by an outlet, which has a lateral or axial pipe and which is referred to as the external outlet, of at least part of the dense phase D1 and having between said opposite ends a length L,
   - at least one internal enclosure of elongated shape along an axis and having a substantially circular cross-section, positioned coaxially with respect to said external enclosure and having a distance Ls, smaller than L, from the extreme end of the external inlet, a so-called internal inlet having a diameter (Di) smaller than (Dc), which is penetrated at least partly by the light phase L1 and at its opposite end recovery means making it possible to recover, by a so-called internal pipe, which is respectively axial if the pipe of the external outlet is lateral, or lateral if the pipe of the external outlet is axial, said part of the light phase L1,
   - said cyclone separator having downstream, in the flow direction of the dense phase D1 from the internal inlet of the internal enclosure, means limiting the advance of the light phase L1 outside said internal enclosure.

**Patentansprüche**

1. Verfahren zur katalytischen Umwandlung einer Charge, die wenigstens eine sauerstoffhaltige Verbindung umfaßt, in olefinische Kohlenwasserstoffe, die reich an Verbindungen mit 2 bis 4 Kohlenstoffatomen in ihrem Molekül sind, die Umwandlung im Schleppbett dieser Charge in einer Umwandlungsreaktionszone länglicher Gestalt unter geeigneten Bedingungen in Anwesenheit eines Katalysators in Form von Feststoffpartikeln umfassend, wobei das Verfahren umfaßt: eine Stufe, bei der in eine Zone, die benachbart einem ersten Ende dieser Reaktionszone angeordnet ist, wenigstens ein Feststoff in Form von katalytische Partikel enthaltenden Partikeln und wenigstens ein Schleppfluid eingespeist wird; eine Zone, bei der, und im Fall einer wenigstens zum Teil flüssigen Charge, diese Charge in eine Einführungszone zerstäubt wird, die in Bewegungsrichtung der Feststoffpartikel stromabwärts zur Einführungszone dieser Feststoffpartikel sich befindet, eingeführt wird, eine Stufe, bei der diese Feststoffpartikel und diese Charge in einer Zone kontaktiert werden, die benachbart diesem ersten Ende sich befindet, eine Zone, bei der die Feststoffpartikel und die Charge in der Reaktionszone in Zirkulation versetzt werden, während der man die Umwandlung dieser Charge durchführt und wenigstens zum Teil diese katalytischen Feststoffpartikel durch Abscheiden von Koks auf diesen desaktiviert, eine Stufe zum Trennen eines Gemisches M1 aus Feststoffpartikeln und Gas, welches die Umwandlungsprodukte, wenigstens teilweise dieser Charge enthält in einer Trennzone, die benachbart einem zweiten Ende der Reaktionszone entgegengesetzt zu diesem ersten Ende sich befindet, eine Regenerationsstufe, in wenigstens einer Reaktionszone, bei der wenigstens ein Teil der katalytischen Feststoffpartikel, die wenigstens zum Teil desaktiviert sind regeneriert werden und eine Stufe zur Recyclierung der katalytischen wenigstens zum Teil regenerierten Feststoffpartikel in eine Rezyklierungszone benachbart diesem ersten Ende, dadurch gekennzeichnet, daß die Trennung der Feststoffpartikel und der die Reaktionsprodukte enthaltenden Gase in einem Zyklon-Gleichstromseparator durchgeführt wird.

2. Verfahren nach Anspruch 1, bei dem der Zyklon-Gleichstromseparator in Kombination umaßt:
   - wenigstens einen äußeren umschlossenen Raum von längs einer Achse länglicher Gestalt und im wesentlichen kreisförmigem Querschnitt vom Durchmesser (Dc), ein erstes Ende der Einführungsmittel umfassend, das es ermöglicht, über einen sogenannten äußeren Eintritt das Gemisch M1 einzuführen, welches eine erste dichte Phase D1 bildende Feststoffpartikel und die eine leichte Phase L1 bildenden Gase enthält, wobei diese Mittel so ausgelegt sind, daß sie wenigstens der leichten Phase L1 eine spiralförmige Bewegung in Strömungsrichtung dieses Gemisches M1 in diesem äußeren umschlossenen Raum erteilen, ebenfalls Mittel zum Trennen der Phasen D1 und L1 umfassend sowie an dem diesem ersten Ende gegenüberliegenden Ende Mittel zur Rückgewinnung, die es ermöglichen, über einen Austritt, der eine seitliche oder axiale Leitung umfaßt, den sogenannten äußeren Austritt wenigstens einen Teil der dichten Phase D1 rückzugewinnen, und der zwischen diesen sich gegenüberliegenden Enden über eine Länge L verfügt,
   - wenigstens einen inneren umschlossenen Raum von längs einer Achse länglicher Gestalt von im wesentlichen kreisförmigem Querschnitt, der koaxial bezogen auf diesen äußeren umschlossenen Raum angeordnet ist, umfassend unter einem Abstand Ls, der kleiner als L ist vom äußersten Niveau des äußeren Eintritts einen sogenannten inneren Eintritt von dem Durchmesser (Di), kleiner (Dc), in den wenigstens ein Teil der leichten Phase L1 eindringt und an seinem gegenüberliegenden Ende Rückgewinnungsmittel, die es ermöglichen, über eine sogenannte innere Leitung, die jeweils axial ist, wenn die Leitung des äußeren Austritts seitlich ist oder die seitlich ist, wenn die Austrittsleitung axial ist, diesen Teil der leichten Phase L1 rückzugewinnen,
   - wobei dieser Zyklonseparator im übrigen abströmseitig des Niveaus des inneren Eintritts des inneren umschlossenen Raums in Zirkulationsrichtung der dichten Phase D1 gesehen, Mittel umfaßt, die die fortschreitende Bewegung der leichten Phase L1 aus diesem inneren umschlossenen Raum hinaus begrenzen.

3. Verfahren nach Anspruch 1 oder 2, bei dem der Gleichstrom-Zyklonseparator wenigstens ein Mittel zum Einführen wenigstens eines Fluides umfaßt, das die Durchführung des Strippens der Feststoffpartikel ermöglicht.

4. Verfahren nach Anspruch 3, bei dem der äußere umschlossene Raum im wesentlichen vertikal ist und die den Fortschritt der leichten Phase L1 aus dem inneren umschlossenen Raum hinaus begrenzenden Mittel im Innern des äußeren umschlossenen Raums und außerhalb des inneren umschlossenen Raums zwischen dem Niveau des inneren Eintritts und den Rückgewinnungsmitteln der dichten Phase D1 positioniert sind.

5. Verfahren nach Anspruch 3, bei dem der äußere umschlossene Raum im wesentlichen horizontal ist und die die fortschreitende Bewegung der leichten Phase L1 nach außen aus dem inneren umschlossenen Raum begrenzenden Mittel stromabwärts in Zirkulationsrichtung der dichten Phase D1 gesehen Rückgewinnungsmittel für die dichte Phase D1 in der Leitung des äußeren Austritts positioniert sind.

6. Verfahren nach einem der Ansprüche 3 bis 5, bei dem die die fortschreitende Bewegung der leichten Phase L1 aus dem inneren umschlossenen Raum begrenzenden Mittel im wesentlichen plane Schaufeln sind, deren Ebene durch eine im wesentlichen vertikale Achse geht.

7. Verfahren nach Anspruch 6, bei dem der Zyklonseparator 2 bis 50 Schaufeln umfaßt, die je eine Abmessung (ep) haben, die gemessen, horizontal, ausgehend von ihrer, der Achse des äußeren umschlossenen Raums am weitesten benachbarten Kante, vom etwa 0,01 bis etwa 1-fachen des Wertes $[((Dc)-(D'e))/2]$ hat, wenn diese Schaufeln im Falle eines Vertikal-Zyklonseparators zwischen der Außenwand des umschlossenen Innenraums vom Außendurchmesser (D'e) und der Innenwand des äußeren umschlossenen Raums vom Innendurchmesser (Dc) positioniert sind, vom etwa 0,01 bis etwa 1-fachen des Wertes (Dc)/2 im Fall eines Vertikal-Zyklonseparators mit seitlichem Innenausgang, wenn sie hinter diesem Innenausgang positioniert sind und vom 0,01 bis 1-fachen des Durchmessers (Ds) der Leitung des äußeren Austritts im Falle eines horizontalen Zyklonseparators eine Abmessung (hpe) hat, die gemessen in der Richtung parallel zur im wesentlichen vertikalen Achse, durch die die Ebene der Schaufel geht, auf der Kante der Schaufel ist, die am weitesten benachbart der Innenwand des äußeren umschlossenen Raumes oder der Innenwand des äußeren Austritts ist und eine Abmessung (hpi) hat, die, gemessen auf der Kante der Schaufel, die am nächsten der Achse des inneren umschlossenen Raums oder der Achse des äußeren Austritts in Richtung parallel zur im wesentlichen parallelen Achse, durch die die Ebene der Schaufel geht, ist, wobei diese Abmessungen (hpe) und (hpi) von etwa 0,1x(Dc) bis etwa 10x(Dc) betragen und diese Schaufeln je unter einem Abstand bezogen auf den inneren Eintritt im Falle eines vertikalen Zyklonseparators oder bezogen auf die Separatormittel im Falle eines horizontalen Zyklonseparators zwischen etwa 0 und etwa 5x(Dc) angeordnet sind.

8. Verfahren nach Anspruch 7, bei dem die Schaufeln je eine Abmessung (hpi) größer oder gleich (hpe) haben.

9. Verfahren nach einem der Ansprüche 2 bis 8, bei dem das Einführungsmittel wenigstens eines eine leichte Phase L2 bildenden Fluids, welches es ermöglicht, gleichzeitig das Strippen der Feststoffpartikel durchzuführen, zwischen dem inneren Eintritt des inneren umschlossenen Raums und dem Ende der Leitung des äußeren Austritts des Zyklonseparators positioniert ist.

10. Verfahren nach einem der Ansprüche 3 bis 9, bei dem der Zyklonseparator zwischen dem äußeren Eintritt und dem inneren Eintritt Mittel zur Stabilisierung der spiralförmigen Strömung wenigstens einer leichten Phase L1 und zur Begrenzung des für die Trennung nützlichen Volumens umfaßt.

11. Verfahren nach einem der Ansprüche 3 bis 10, bei dem das Gemisch M1 gemäß einer Richtung im wesentlichen parallel zur Achse des äußeren umschlossenen Raums oder in einer Richtung im wesentlichen senkrecht zur Achse des äußeren umschlossenen Raums eingeführt wird.

12. Verfahren nach einem der Ansprüche 3 bis 11, bei dem der Zyklonseparator Mittel zur Begrenzung der Störungen zwischen der Strömung des eingeführten Gemisches M1 und den Strömungen der bereits im Separator vorhandenen Phasen umfaßt, die gewählt sind aus einem abfallenden Dach, einer Außenvolute und einer Innenvolute.

13. Verfahren nach einem der Ansprüche 1 bis 12, bei dem die Charge wenigstens einen Alkohol, bevorzugt Methanol, umfaßt, und bei dem sie im gasförmigen Zustand eingeführt wird.

14. Vorrichtung zur katalytischen Umwandlung, im Schleppbett, einer wenigstens eine sauerstoffhaltige Verbindung enthaltenden Charge in olefinische Kohlenwasserstoffe, die reich an Verbindungen sind, welche 2 bis 4 Kohlenstoffatome in ihrem Molekül haben, einen umschlossenen Raum länglicher Gestalt umfassend, in dem diese Umwandlung unter geeigneten Bedingungen durchgeführt wird, der an einem ersten Ende von der Anströmseite zur Abströmseite in Bewegungsrichtung der Charge gesehen wenigstens ein Mittel zum Einführen wenigstens eines Schleppfluids, wenigstens ein Mittel zum Einführen wenigstens eines katalytische Partikel enthaltenden Feststoffs und wenigstens ein Mittel zum Einführen dieser Charge umfaßt, wobei die Vorrichtung benachbart einem zweiten Ende dieses umschlossenen Raums wenigstens ein mit diesem umschlossenen Raum verbundenes Mittel zum Trennen der Feststoffpartikel und der diese Produkte der wenigstens teilweisen Umwandlung dieser Charge und wenigstens ein Mittel zum Regenerieren wenigstens eines Teils der festen katalytischen Charge umfaßt, das einerseits mit dem Mittel zum Abtrennen dieser Feststoffpartikel und andererseits mit diesem umschlossenen Raum benachbart diesem ersten Ende derart verbunden ist, daß die Regenerierung wenigstens eines Teils der katalytischen Feststoffpartikel und die Rezyklierung dieser Feststoffpartikel in diesen umschlossenen Raum ermöglicht wird, dadurch gekennzeichnet, daß dieses Mittel zum Trennen der Feststoffpartikel und der Gase ein im Gleichstrom arbeitender Zyklonseparator ist.

15. Vorrichtung nach Anspruch 14, bei dem der Gleichstrom-Zyklonseparator in Kombination umfaßt:

   - wenigstens einen umschlossenen äußeren Raum von längs einer Achse länglicher Gestalt mit einem im wesentlichen kreisförmigen Querschnitt vom Durchmesser (Dc), der an einem ersten Ende Einführungsmittel umfaßt, die es ermöglichen, über einen Eintritt, den sogenannten äußeren Eintritt das Gemisch M1 einzuführen, welches die die dichte Phase D1 bildenden Feststoffpartikel sowie die die leichte Phase L1 bildenden Gase enthält, wobei diese Mittel so ausgelegt sind, daß sie wenigstens der leichten Phase L1 eine spiralförmige Bewegung in Strömungsrichtung dieses Gemisches M1 in diesem äußeren umschlossenen Raum erteilen und ebenfalls Mittel zum Trennen der Phasen D1 und L1 umfassen und an dem diesem ersten Ende gegenüberliegenden Ende Rückgewinnungsmittel aufweist, die es ermöglichen, über einen Ausgang, der eine seitliche oder axiale Leitung, die sogenannte Austrittsleitung, umfaßt, wenigstens einen Teil der dichten Phase D1 zurückzugewinnen, wobei erstere zwischen diesen gegenüberliegenden Wänden eine Länge L hat,

   - wenigstens einen inneren umschlossenen Raum von längs einer Achse länglicher Gestalt mit im wesentlichen kreisförmigem Querschnitt, der koaxial bezogen auf diesen äußeren umschlossenen Raum angeordnet ist und unter einer Entfernung Ls, die kleiner als L ist vom äußersten Niveau des äußeren Eintritts einen Eingang, den sogenannten inneren Eingang vom Durchmesser (Di), kleiner (Dc), umfaßt, in den wenigstens ein Teil der leichten Phase L1 eindringt und an ihrem gegenüberliegenden Ende Rückgewinnungsmittel, die es ermöglichen, über eine sogenannte Innenleitung, die jeweils axial ist, wenn die Leitung des äußeren Ausgangs seitlich ist oder seitlich ist, wenn die Leitung des äußeren Ausgangs axial ist, diesen Teil der leichten Phase L1 rückzugewinnen, umfaßt,

   - wobei der Zyklonseparator im übrigen abströmseitig in Zirkulationsrichtung der dichten Phase D1 vom Niveau des inneren Eintritts des inneren umschlossenen Raums gesehen Mittel aufweist, die das allmähliche Fortschreiten der leichten Phase L1 von diesem inneren umschlossenen Raum nach außen begrenzen.

**FIG.2A**

**FIG.2B**

**FIG.1**

## FIG.3

EP 0 498 726 B1

FIG.4

19

## FIG.5

## FIG.6